(19) Europäisches Patentamt
European Patent Office
Office européen des brevets

(11) Numéro de publication: **0 261 035 B1**

(12) **FASCICULE DE BREVET EUROPEEN**

(45) Date de publication de fascicule du brevet: **11.03.92**

(51) Int. Cl.[5]: **C07D 209/18**, A01N 53/00

(21) Numéro de dépôt: **87402085.2**

(22) Date de dépôt: **18.09.87**

Le dossier contient des informations techniques présentées postérieurement au dépôt de la demande et ne figurant pas dans le présent fascicule.

(54) **Dérivés de l'indole, leur procédé de préparation, leur application à la lutte contre les parasites et les compositions les renfermant.**

(30) Priorité: **18.09.86 FR 8613051**

(43) Date de publication de la demande:
**23.03.88 Bulletin 88/12**

(45) Mention de la délivrance du brevet:
**11.03.92 Bulletin 92/11**

(84) Etats contractants désignés:
**BE CH DE ES FR GB GR IT LI NL**

(56) Documents cités:
**EP-A- 0 176 387**
**FR-A- 2 218 316**

**The Chemistry of Heterocyclic Compounds, (1979), tome 25, pages 170-177**

(73) Titulaire: **ROUSSEL-UCLAF**
**35, Boulevard des Invalides**
**F-75007 Paris(FR)**

(72) Inventeur: **Tessier, Jean**
**30, rue Jean Moulin**
**F-94300 Vincennes(FR)**
Inventeur: **Demassey, Jacques**
**Allée Saint Jacques, Chalifert**
**F-77144 Montevrain(FR)**
Inventeur: **Demoute, Jean-Pierre**
**249bis, rue de Rosny**
**F-93100 Montreuil-Sous-Bois(FR)**

(74) Mandataire: **Tonnellier, Marie-José et al**
**Département des Brevets ROUSSEL UCLAF**
**B.P. no 9**
**F-93230 Romainville(FR)**

**EP 0 261 035 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne de nouveaux dérivés de l'indole, leur procédé de préparation, leur application à la lutte contre les parasites et les compositions les renfermant.

On connaissait déjà les dérivés du pyrrole doués d'activité pesticide (cf EP 0176 387), on vient de découvrir de nouveaux dérivés de l'indole présentant d'intéressantes propriétés pesticides. L'invention a pour objet les composés de formule (I) :

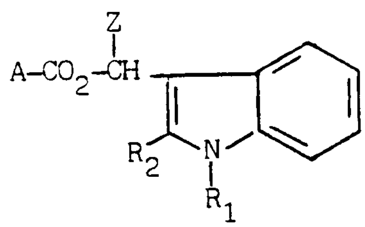

(I)

dans laquelle Z représente un atome d'hydrogène, un groupement C≡N, C≡CH, $CF_3$ ou un radical alkyle renfermant de 1 à 3 atomes de carbone, $R_1$ représente un atome d'hydrogène, un radical alkyle, linéaire, ramifié, cyclique, saturé ou insaturé renfermant jusqu'à 8 atomes de carbone, un radical phényle ou naphtyle ou un radical benzyle, phényl éthyle, phényl propyle, phényl butyle, phényl pentyle ou phényl hexyle, et $R_2$ représente un atome d'hydrogène, un atome d'halogène, un radical alkyle, linéaire, ramifié ou cyclique, saturé ou insaturé, renfermant jusqu'à 8 atomes de carbone, un radical phényle ou napthyle, un radical benzyle, phényl éthyle, phényl propyle, phényl butyle, phényl pentyle, phényl hexyle, un radical cyano, un radical $CF_3$, un radical $CO_2$alkyle renfermant jusqu'à 18 atomes de carbone, un radical $NO_2$ et A représente un radical :

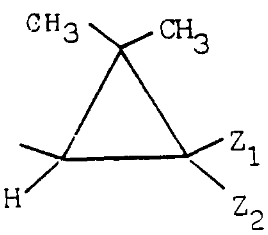

dans lequel $Z_1$ représente un atome d'hydrogène et $Z_2$ représente un radical :

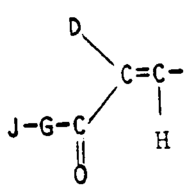

dans lequel D représente un atome d'hydrogène ou d'halogène, un radical alkoxyle renfermant de 1 à 8 atomes de carbone, G représente un atome d'oxygène ou de soufre et J représente un radical alkyle linéaire, ramifié ou cyclique, saturé ou insaturé, renfermant de 1 à 8 atomes de carbone, éventuellement substitué par un ou plusieurs atomes d'halogène lorsque D est un atome d'hydrogène.

Lorsque Z représente un radical alkyle, il s'agit de préférence d'un radical méthyle.

Lorsque $R_1$ ou $R_2$ représente un radical alkyle, linéaire ou ramifié, il s'agit de préférence des radicaux méthyle, éthyle, propyle linéaire ou ramifié, butyle linéaire ou ramifié, pentyle linéaire ou ramifié, hexyle linéaire ou ramifié, heptyle linéaire ou ramifié, octyle linéaire ou ramifié.

Lorsque $R_1$ ou $R_2$ représente un radical alcoyle insaturé, il s'agit de préférence des radicaux allyle, 1-propényle, 2-propényle, 1-(2-propynyle).

Lorsque $R_1$ ou $R_2$ représente un radical alkyle cyclique, il s'agit des radicaux cyclopropyle, cyclobutyle,

cyclopentyle, cyclohexyle.

L'invention a tout particulièrement pour objet les composés de formule I dans lesquels $R_1$ représente un radical $-CH_2-C{\equiv}CH$, un radical $CH_2-C_6H_5$ ou un atome d'hydrogène.

L'invention a tout particulièrement pour objet les composés de formule I dans lesquels $R_2$ représente un radical $CF_3$.

Parmi les composés préférés de l'invention on peut citer ceux dans lesquels A représente un radical :

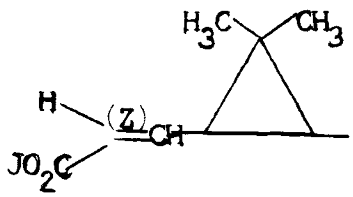

dans lequel J représente un radical alkyle renfermant de 1 à 8 atomes de carbone, linéaire, ramifié ou cyclisé et saturé éventuellement substitué par un ou plusieurs atomes d'halogène $Hal_2$, la double liaison ayant la géométrie Z ainsi que ceux dans lesquels A représente un radical :

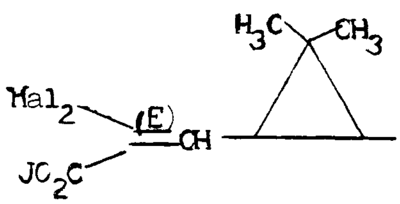

dans lequel $Hal_2$ représente un atome d'halogène et J représente un radical alkyle renfermant de 1 à 8 atomes de carbone, linéaire, ramifié ou cyclisé et saturé, la double liaison ayant la géométrie (E).

Comme valeur préférée de $Hal_2$, on peut citer le fluor.

L'invention a naturellement particulièrement pour objet les composés dont la préparation est donnée dans la partie expérimentale et tout spécialement le :

(1R,3S)-2,2-diméthyl-3-[(Z)-3-oxo-3-[2,2,2-trifluorol-1-(trifluorométhyl) éthoxy]-1-propényl]-cyclopropanecarboxylate de [1-(2-propynyl)-2-triluorométhyl-(1H)-indol]-3-yl] méthyle.

L'invention a également pour objet un procédé de préparation des composés de formule I, caractérisé en ce que l'on soumet un alcool de formule (II) :

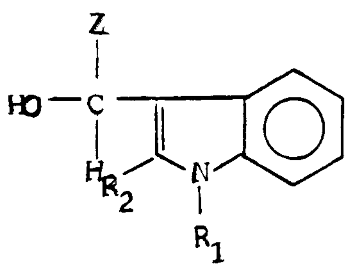

(II)

dans laquelle Z, $R_1$ et $R_2$ ont la signification indiquée précédemment à l'action d'un acide de formule (III) :

$ACO_2H$ (III)

dans laquelle A a la signification indiquée précédemment ou d'un dérivé fonctionnel de cet acide et obtient le composé de formule I correspondant.

Dans un mode de réalisation préférée de l'invention, on fait réagir l'acide III et l'alcool II en présence de dicyclohexylcarbodiimide et de 4-diméthyl aminopyridine.

Les alcools de formule II peuvent être préparés à partir de l'indole et des dérivés substitués de l'indole comme il est indiqué ci-après dans la partie expérimentale.

Les composés de formule (I) présentent d'intéressantes propriétés qui permettent leur utilisation dans la

lutte contre les parasites.

Il peut s'agir par exemple de la lutte contre les parasites des végétaux, les parasites des locaux et les parasites des animaux à sang chaud. C'est ainsi que l'on peut utiliser les produits de l'invention pour lutter contre les insectes, les nématodes et les acariens parasites des végétaux et des animaux.

L'invention a donc pour objet l'application des composés de formule I à la lutte contre les parasites des végétaux, les parasites des locaux et les parasites des animaux à sang chaud.

Les produits de formule (I) peuvent être utilisés notamment pour lutter contre les insectes dans le domaine agricole, pour lutter par exemple contre les pucerons, les larves de lépidoptères et les coléoptères. Ils sont utilisés à des doses comprises entre 10 g et 300 g de matière active à l'hectare.

Les produits de formule (I) peuvent également être utilisés pour lutter contre les insectes dans les locaux, pour lutter notamment contre les mouches, les moustiques et les blattes.

Les produits de formule (I) sont de plus photostables et ne sont pas toxiques pour les mammifères.

L'ensemble de ces propriétés fait des produits de formule (I) des produits qui correspondent parfaitement aux exigences de l'industrie agrochimique moderne : ils permettent de protéger les récoltes tout en préservant l'environnement.

Les produits de formule (I) peuvent aussi être utilisés pour lutter contre les acariens et les nématodes parasites des végétaux.

Les composés de formule (I) peuvent encore être utilisés pour lutter contre les acariens parasites des animaux, pour lutter par exemple contre les tiques et notamment les tiques de l'espèce Boophilus, ceux de l'espèce Hyalomnia, ceux de l'espèce Amblyomnia et ceux de l'espèce Rhipicephalus ou pour lutter contre toutes sortes de gales et notamment la gale sarcoptique, la gale psoroptique et la gale chorioptique.

L'invention a donc notamment pour objet les compositions destinées à la lutte contre les parasites des végétaux, les parasites des locaux et les parasites des animaux à sang chaud, caractérisées en ce qu'elles renferment comme principe actif au moins un des produits définis ci-dessus et plus particulièrement le produit dont la nomenclature est indiquée ci-dessus.

L'invention a tout spécialement pour objet les compositions acaricides pour lutter contre les acariens parasites des végétaux contenant comme matière active un composé de formule I. L'activité acaricide des produits de l'invention est en effet remarquable comme le montrent les tests de la partie expérimentale.

L'invention a aussi pour objet les compositions insecticides renfermant comme principe actif au moins un des produits définis ci-dessus.

L'invention a encore pour objet les compositions nématicides renfermant comme principe actif au moins un des produits définis ci-dessus.

L'invention a également pour objet les compositions acaricides destinées à la lutte contre les parasites des animaux à sang chaud, caractérisées en ce qu'elles renferment comme principe actif, au moins un des produits définis ci-dessus.

Les compositions de l'invention sont préparées selon les procédés usuels de l'industrie agrochimique ou de l'industrie vétérinaire ou de l'industrie des produits destinés à la nutrition animale.

Ces compositions peuvent se présenter sous forme de poudres, granulés, suspensions, émulsions, solutions, solutions pour aérosols, bandes combustibles, appâts ou autres préparations employés classiquement pour l'utilisation de ce genre de composés.

Outre le principe actif, ces compositions contiennent, en général, un véhicule et/ou un agent tensioactif, non ionique, assurant, en outre, une dispersion uniforme des substances constitutives du mélange. Le véhicule utilisé peut être un liquide, tel que l'eau, l'alcool, les hydrocarbures ou autres solvants organiques, une huile minérale, animale ou végétale, une poudre telle que le talc, les argiles, les silicates, le kieselguhr ou un solide combustible.

Les compositions insecticides selon l'invention contiennent de préférence de 0,005 % à 10 % en poids de matière active.

Selon un mode opératoire avantageux, pour un usage dans les locaux, les compositions selon l'invention sont utilisées sous forme de compositions fumigantes.

Les compositions insecticides selon l'invention peuvent alors être avantageusement constituées, pour la partie non active, d'un serpentin insecticide (ou coil) combustible, ou encore d'un substrat fibreux incombustible. Dans ce dernier cas, le fumigant obtenu après incorporation de la matière active est placé sur un appareil chauffant tel qu'un électromosquito destroyer.

Dans le cas où l'on utilise un serpentin insecticide, le support inerte peut être, par exemple, composé de marc de pyrèthre, poudre de Tabu (ou poudre de feuilles Machilus Thumbergii), poudre de tige de pyrèthre, poudre de feuille de cèdre, poudre de bois (telle que de la sciure de pin) amidon et poudre de coque de noix de coco.

La dose de matière active peut alors être, par exemple, de 0,03 à 1 % en poids.

Dans le cas où l'on utilise un support fibreux incombustible, la dose de matière active peut alors être, par exemple, de 0,33 à 95 % en poids.

Les compositions selon l'invention pour un usage dans les locaux peuvent aussi être obtenues en préparant une huile pulvérisable à base de principe actif, cette huile imbibant la mèche d'une lampe et étant alors soumise à la combustion.

La concentration du principe actif incorporé à l'huile est, de préférence, de 0,03 à 95 % en poids.

Les compositions insecticides selon l'invention, comme les compositions acaricides et nématicides peuvent être additionnées éventuellement d'un ou plusieurs autres agents pesticides. Les compositions acaricides et nématicides peuvent se présenter notamment sous forme de poudres, granulés, suspensions, émulsions, solutions.

Pour l'usage acaricide sur végétaux, on utilise de préférence des poudres mouillables, pour pulvérisation foliaire, contenant de 1 à 80 % ou des liquides pour pulvérisation foliaire contenant de 1 à 500 g/l de principe actif. On peut également employer des poudres pour poudrages foliaires contenant de 0,05 à 3 % de matière active.

Pour l'usage nématicide, on utilise de préférence des liquides pour traitement des sols contenant de 300 à 500 g/l de principe actif.

Les composés acaricides et nématicides selon l'invention sont utilisés, de préférence, à des doses comprises entre 1 et 100 g de matière active à l'hectare.

Lorsqu'il s'agit de lutter contre les acariens parasites des animaux, on incorpore très souvent les produits de l'invention dans des compositions alimentaires en association avec un mélange nutritif adapté à l'alimentation animale. Le mélange nutritif peut varier selon l'espèce animale, il peut renfermer des céréales, des sucres et des grains, des tourteaux de soja, d'arachide et de tournesol, des farines d'origine animale, par exemple, des farines de poissons, des acides aminés de synthèse, des sels minéraux, des vitamines et des anti-oxydants.

Les composés de formule (I) présentent une excellente tolérance générale et l'invention a donc également pour objet des produits de formule (I) pour lutter notamment contre les affections créees par les poux, les tiques et les gales.

Les produits de l'invention peuvent être administrés par voie externe, par vaporisation, par shampooing, par bain ou badigeonnage.

Les produits de l'invention à usage vétérinaire peuvent être également administrés par badigeonnage de l'épine dorsale selon la méthode dite méthode "pour-on".

L'invention a aussi pour objet les associations douées d'activité insecticide, acaricide ou nématicide, caractérisées en ce qu'elles contiennent comme matière active d'une part un au moins des composés de formule générale (I) telle que définie ci-dessus et d'autre part, un au moins des esters pyréthrinoïdes choisis dans le groupe constitué par les esters d'alléthrolone, d'alcool 3,4,5,6-tétrahydrophtalimido méthylique, d'alcool 5-benzyl 3-furyl méthylique, d'alcool 3-phénoxy benzylique et d'alcools alpha-cyano 3-phénoxy benzyliques des acides chrysanthémiques, par les esters d'alcool 5-benzyl 3-furyl méthylique des acides 2,2-diméthyl 3-(2-oxo 3-tétrahydrothiophénylidène méthyl) cyclopropane-1-carboxyliques, par les esters d'alcool 3-phénoxy benzylique et d'alcools alpha-cyano 3-phénoxy benzyliques des acides 2,2-diméthyl 3-(2,2-dichlorovinyl) cyclopropane-1-carboxyliques, par les esters d'alcools alpha-cyano 3-phénoxy benzyliques d'acides 2,2-diméthyl 3-(2,2-dibromovinyl) cyclopropane-1-carboxyliques, par les esters d'alcool 3-phénoxy benzylique des acides 2-parachlorophényl 2-isopropyl acétiques, par les esters d'alléthrolone, d'alcool 3,4,5,6-tétrahydrophtalimido méthylique, d'alcool 5-benzyl 3-furyl méthylique, d'alcool 3-phénoxy benzylique et d'alcools alpha-cyano 3-phénoxybenzyliques des acides 2,2-diméthyl 3-(1,2,2,2-tétrahaloèthyl) cyclopropane-1-carboxyliques, dans lesquels "halo" représente un atome de fluor, de chlore ou de brome, étant entendu que les copules acides et alcools des esters pyréthrinoïdes ci-dessus peuvent exister sous toutes leurs formes stéréoisomères possibles.

Pour exalter l'activité biologique des produits de l'invention, on peut les additionner à des synergistes classiques utilisés en pareil cas tel que le 1-(2,5,8-trioxadodécyl) 2-propyl 4,5-méthylènedioxy benzène (ou butoxyde de pipéronyle) ou la N-(2-éthyl heptyl) bicyclo[2.2.1]-5- heptène-2,3-di carboximide ou le pipéronyl bis 2-(2'-n-butoxy éthoxy) éthyl acétal (ou tropital).

L'invention a également pour objet les compositions telles que définies ci-dessus caractérisées en ce qu'elles renferment en outre un synergiste des pyréthrinoïdes.

Les exemples suivants illustrent l'invention sans toutefois la limiter.

Préparation 1: Le (2-trifluorométhyl (1H) indol-3-yl) méthanol utilisé ci-aprés peut être préparé de la manière suivante :

**Stade A :** 2-trifluorométhyl 3-formyl (1H) indole.

On mélange à -65¤C, sous atmosphère inerte, 6,1 g de chlorure d'aluminium, 20 cm3 de chlorure de méthylène, introduit à -60¤C, une solution de 4,61 g de 2-trifluorométhyl (1H) indole (J.Org.Chem. 1974 39, 1836), 1,4 cm3 de nitrométhane et 20 cm3 de chlorure de méthylène, ajoute à la suspension obtenue 3,3 cm3 de dichlorométhyl éther en solution dans 10 cm3 de chlorure de méthylène, agite 15 minutes à -65¤C, laisse remonter la température, observe vers -10¤C un vif dégagement d'acide chlorhydrique qui cesse vers 0¤C, agite la solution noire obtenue pendant 30 minutes à 25¤C, verse le mélange réactionnel dans l'eau, extrait au chlorure de méthylène, lave les phases organiques avec une solution aqueuse de bicarbonate de sodium, puis à l'eau, concentre à sec sous pression réduite et obtient 4,6 g de 2-trifluorométhyl 3-formyl (1H) indole.

**Stades B :** (2-trifluorométhyl (1H) indol-3-yl) méthanol.

On introduit à +5¤C par petites fractions 2 g de borohydrure de potassium dans une solution comprenant 4,6 g de produit obtenu au stade A, 150 cm3 de tétrahydrofuranne et 30 cm3 d'eau et agite une heure. On laisse revenir à température ambiante, élimine le tétrahydrofuranne sous pression réduite, dilue avec de l'éther, lave à l'eau, sèche et concentre sous pression réduite; on chromatographie le résidu sur silice (éluant hexane-acétate d'éthyle 7-3) et obtient 3 g de produit attendu. F = 106¤C.

**Exemple préliminaire P : (1R,3S) 3-(2,2-dibromoéthényl) 2,2-diméthyl cyclopropane carboxylate de [2-trifluorométhyl 1-(2-propynyl) (1H) indol-3-yl] méthyle.**

On mélange sous atmosphère inerte, 1 g de 1-(2-propynyl) 2-trifluorométhyle (1H) indol-3-yl) méthanol, 10 cm3 de chlorure de méthylène et une solution de 1,4 g d'acide 3-(2,2-dibromoéthényl) 2,2-diméthyl cyclopropane carboxylique dans 10 cm3 de chlorure de méthylène, introduit à 0¤C, 1,05 g de dicyclohexylcarbodiimide et 100 mg de 4-diméthylamino pyridine dans 8 cm3 de chlorure de méthylène, agite 15 minutes à 0¤C puis une heure à 20¤C, on filtre, concentre à sec le filtrat par distillation sous pression réduite, ajoute de l'éther isopropylique, filtre, concentre, chromatographie le résidu sur silice en éluant par un mélange d'hexane et d'éther isopropylique (9/1) et obtient 1,8 g d'ester attendu. F = 60¤C.
$[alpha]_D$ = -17¤ + 2¤ (c = 0,5 %, $CHCl_3$)

| Analyse : $C_{21}H_{18}Br_2F_3NO_2$ 533,196 | | | | | |
|---|---|---|---|---|---|
| Calculé :<br>Trouvé : | C% 47,31<br>47,3 | H% 3,40<br>3,4 | Br% 29,97<br>29,8 | F% 10,69<br>10,9 | N% 2,63<br>2,7 |

Préparation 2: Le (1-(2-propynyl) 2-trifluorométhyl (1H) indol-3-yl) méthanol utilisé ci-aprés peut être préparé comme suit.

**Stade A :** 1-(2-propynyl) 2-trifluorométhyl (1H) indole.

On mélange sous atmosphère inerte à +5¤C, 8,57 g de 2-trifluorométhyl 1H indole décrit dans J.Org. Chem. (1983) 48,3233, 100 cm3 de tétrahydrofuranne, ajoute en 15 minutes, 2,4 g d'hydrure de sodium dans l'huile, introduit en 2 minutes 4,5 cm3 de 2-bromo propyne, agite 30 minutes à +5¤C puis une heure à 25¤C, ajoute de nouveau 4,5 cm3 de bromo propyne et agite pendant 4 heures, verse sur une solution glacée de phosphate monosodique, extrait à l'éther éthylique, concentre à sec par distillation sous pression réduite, chromatographie le résidu sur silice en éluant par un mélange d'hexane et d'acétate d'éthyle (95/5) et obtient 8,88 g de produit recherché. F = 59¤C.

**Stade B :** 1-(2-propynyl) 2-trifluorométhyl 3-formyl (1H) indole

De manière analogue à celle utilisée au Stade A de la préparation 1, mais en utilisant 8,8 g de 1-(2-propynyl) 2-trifluorométhyl (1H) indole, on obtient 6,70 g de produit recherché. F = 106¤C.

**Stade C :** (1-(2-propynyl) 2-trifluorométhyl (1H) indol-3-yl) méthanol.

On mélange à +5¤C, 5,54 g de 1-(2-propynyl) 2-trifluorométhyl 3-formyl (1H) indole, 120 cm3 de tétrahydrofurane, 20 cm3 d'eau, ajoute en petites fractions, 2 g de borohydrure de potassium, concentre à faible volume par distillation sous pression réduite, ajoute de l'éther éthylique, lave la phase organique à l'eau salée, concentre à sec par distillation sous pression réduite et obtient 5,6 g de produit recherché. F = 90¤C.

Exemple 1 :

(1R,3S)-3-((E)-3-éthoxy-2-fluoro-3-oxo-1-propényl) -2,2-diméthyl-cyclopropanecarboxylate de [1-(2-propynyl)-2-trifluorométhyl-(1H)-indol-3-yl]méthyle

En opérant de manière analogue à celle de l'exemple P, au départ de 1 g d'alcool et de 1,1 g d'acide correspondant, on obtient 1,84 g d'ester attendu.
[alpha]$_D$ = +12¤ ± 2¤ (c = 0,5 % $CHCl_3$)

| Analyse : $C_{24}H_{23}F_4NO_4$ 465,445 | | | | |
|---|---|---|---|---|
| Calculé :<br>Trouvé : | C% 61,93<br>62,0 | H% 4,98<br>5,0 | N% 3,01<br>3,3 | F% 16,33<br>16,3. |

Exemple 2 :

(1R,3S)-3-[(Z)-3-(1,1-diméthylyéthoxy)-3-oxo-1-propényl] -2,2-diméthyl-cyclopropanecarboxylate de [2-trifluorométhyl-(1H)-indol-3-yl]méthyle

De manière analogue à celle de l'exemple P au départ de 1 g d'alcool et de 1,2 g d'acide correspondant, on obtient 0,84 g d'ester désiré.
[alpha]$_D$ = + 38¤ ± 2¤ (c = 0,5 %, $CHCl_3$)

| Analyse : $C_{23}H_{26}F_3NO_4$ 437,463 | | | | |
|---|---|---|---|---|
| Calculé :<br>Trouvé : | C% 63,15<br>63,1 | H% 5,99<br>6,1 | N% 3,20<br>3,1 | F% 13,03<br>13,3. |

Exemple 3 :

(1R,3S)-3-[(Z)-3-(1,1-diméthyléthoxy)-3-oxo-1-propényl] -2,2-diméthyl-cyclopropanecarboxylate de [1-(2-propynyl)-2-trifluorométhyl-(1H)-indol-3-yl]méthyl

De manière analogue à celle de l'exemple P, au départ de 1 g d'alcool et de 1,15 g d'acide correspondant, on obtient 1,67 g d'ester attendu. F = 60¤C.
[alpha]$_D$ = + 33,5¤ ± 2¤ (c = 0,5 %, $CHCl_3$)

| Analyse : $C_{26}H_{28}F_3NO_4$ 475,513 | | | | |
|---|---|---|---|---|
| Calculé :<br>Trouvé : | C% 65,67<br>65,9 | H% 5,94<br>6,0 | F% 11,99<br>11,9 | N% 2,95<br>2,9 |

Exemple 4 :

(1R,3S)-3-[(Z)-3-méthoxy-3-oxo-1-propényl]-2,2-diméthyl-cyclopropanecarboxylate de [1-(2-propynyl)-2-trifluorométhyl-(1H)-indol-3-yl]méthyle

De manière analogue à celle de l'exemple P, au départ de 1 g d'alcool et de 0,870 g d'acide

correspondant, on obtient 1,7 g d'ester recherché. F = 66¤C.
$[alpha]_D$ = + 28¤ ± 1¤ (c = 1%, $CHCl_3$)

| Analyse : $C_{23}H_{22}F_3NO_4$ 433,431 | | | | |
|---|---|---|---|---|
| Calculé :<br>Trouvé : | C% 63,74<br>63,9 | H% 5,17<br>5,1 | N% 3,23<br>3,2 | F% 13,15<br>13,0. |

Exemple 5 :

(1R,3S)-3-[(E)-3-(1,1-diméthyléthoxy)-2-fluoro-3-oxo-1-propényl] -2,2-diméthyl-cyclopropanecarboxylate de [1-(2-propynyl)-2-trifluorométhyl-(1H)-indol-3-yl] méthyle

De manière analogue à celle de l'exemple P, au départ de 1,48 g d'alcool et de 1,54 g d'acide correspondant, on obtient 2,03 g d'ester recherché.
$[alpha]_D$ = -10,5¤ + 1¤ (c = 1%, toluène)

| Analyse : $C_{26}H_{27}F_4NO_4$ 493,504 | | | | |
|---|---|---|---|---|
| Calculé :<br>Trouvé : | C% 63,28<br>63,5 | H% 5,51<br>5,6 | N% 2,84<br>2,7 | F% 15,40<br>15,5. |

Préparation 3 :

Le (1-benzyl 2-trifluorométhyl (1H) indol-3-yl) méthanol

**Stade A :** 1-benzyl 2-trifluorométhyl (1H) indole.

On mélange sous atmosphère inerte 8,6 g de 2-trifluorométhyl (1H) indole, 100 cm3 de tétrahydrofuranne, refroidit à 0¤C, ajoute par petites fractions en 15 minutes environ 2,4 g d'une suspension d'hydrure de sodium à 50 % dans l'huile, introduit en 5 minutes, 6,5 cm3 de bromure de benzyle en solution dans 10 cm3 de tétrahydrofuranne, agite pendant 42 heures à température ambiante, verse le mélange réactionnel dans de l'eau saturée de phosphate monosodique, extrait à l'éther éthylique, sèche, concentre à sec par distillation sous pression réduite, chromatographie le résidu sur silice en éluant par un mélange d'hexane et d'acétate d'éthyle (9/1) et obtient 12,54 g de produit recherché. F = 20¤C.

**Stade B :** 1-benzyl 2-trifluorométhyl 3-formyl (1H) indole.

En opérant de façon analogue à celle indiquée au stade A de la préparation 1 mais en utilisant 11,07 g de 1-benzyl 2-trifluorométhyl (1H) indole, on obtient 7,94 g de produit recherché.

**Stade C :** (1-benzyl 2-trifluorométhyl (1H) indol-3-yl) méthanol.

En opérant de façon analogue à celle indiquée au stade B de la préparation 1 mais en utilisant 7,9 g de 1-benzyl 2-trifluorométhyl 3-formyl (1H) indole préparé ci-dessus, on obtient 7,45 g de produit recherché. F = 96¤.

Exemple 6 :

(1R,3S)-3-[(E)-3-(1,1-diméthyléthoxy)-2-fluoro-3-oxo-1-propényl]-2,2-diméthyl-cyclopropanecarboxylate de [1-(phénylméthyl)-2-trifluorométhyl-(1H)-indol-3-yl]méthyle

En opérant comme précédemment, au départ de 1,52 g d'alcool et de 1,33 g d'acide correspondant, on obtient 1,90 g d'ester recherché.
$[alpha]_D$ = +4¤ ± 1¤ (c = 0,5 %, toluène)

| Analyse : $C_{30}H_{31}F_4NO_4$ 545,58 | | | | |
|---|---|---|---|---|
| Calculé :<br>Trouvé : | C% 66,05<br>66,2 | H% 5,73<br>5,7 | N% 2,57<br>2,6 | F% 13,93<br>13,9. |

Exemple 7 :

(1R,3S)-3-[(E)-3-(1,1-diméthyléthoxy)-2-fluoro-3-oxo-1-propényl] -2,2-diméthyl-cyclopropanecarboxylate de cyano[1-(2-propynyl)-2-trifluorométhyl-(1H)-indol-3-yl]méthyle

En opérant comme précédemment au départ de 0,65 g de cyanohydrine et de 0,6 g d'acide correspondant, on obtient 0,73 g d'ester recherché.
$[alpha]_D$ = +25¤ ± 1¤ (c = 0,8 %, toluène)

| Analyse : $C_{27}H_{26}N_2F_4O_4$ 518,519 | | | | |
|---|---|---|---|---|
| Calculé :<br>Trouvé : | C% 62,54<br>62,5 | H% 5,05<br>4,9 | N% 5,40<br>5,6 | F% 14,66<br>14,8 |

L'alcool utilisé dans l'exemple 7 peut être préparé selon la demande de brevet néerlandais 65-17259 publiée le 1er Juillet 1966.

Exemple 8 :

(1R,3S)-2,2-diméthyl-3-[(Z)-3-oxo-3-[2,2,2-trifluoro -1-(triofluorométhyl)éthoxy]-1-propényl]-cyclopropanecarboxylate de [1-(2-propynyl)-2-trifluorométhyl-(1H)-indol-3-yl]méthyle

On mélange sous atmosphère inerte 1,35 g d'acide préparé comme indiqué dans le brevet européen n¤ 0 048 186, 4 cm3 d'une solution de dichlorométhane contenant 1 g de [1-(2-propynyl) 2-trifluorométhyl (1H) indol-3-yl] méthanol et 16 cm3 de dichlorométhane puis refroidit à -10¤C et ajoute en 5 minutes 838 mg de dicyclohexylcarbodiimide, 46 mg de diméthylaminopyridine dans 10 cm3 de dichlorométhane. On maintient l'agitation pendant 1 heure à -10¤C/-5¤C puis essore, concentre à sec sous pression réduite, chromatographie le résidu sur silice (éluant : hexane-acétate d'éthyle 9-1) et recueille 1,58 g de produit attendu (isomère delta Z). F = 92¤C.
$[alpha]_D$ = -13¤ ± 1,5¤ (c = 0,7%, toluène)

| Analyse : $C_{25}H_{20}F_9NO_4$ : 569,429 | | | | |
|---|---|---|---|---|
| Calculé :<br>Trouvé : | C% 52,73<br>52,9 | H% 3,54<br>3,6 | N% 2,46<br>2,5 | F% 30,03<br>29,9 |

Exemple 9 :

(1R,3S)-2,2-diméthyl-3-[(E)-3-oxo-3-[2,2,2-trifluoro -1-(trifluorométhyl)éthoxy]-1-propényl] -cyclopropanecarboxylate de [1-(2-propynyl)-2-trifluorométhyl-(1H)-indol-3-yl]méthyle

On opère comme à l'exemple 8 au départ de 1,1 g de l'alcool utilisé à l'exemple 8 et 1,45 g de l'acide utilisé à l'exemple 8 mais en maintenant le milieu réactionnel sous agitation 1 heure et demie à +5¤/+10¤C puis 1 heure et demie à température ambiante. On obtient 1,58 g de produit attendu (isomère delta E) que l'on purifie par chromatographie sur silice (éluant : hexane-éther isopropylique 7-3). F = 92¤C.
$[alpha]_D$ = -49,5¤ ± 2¤ (c = 0,6%, toluène)

| Analyse : $C_{25}H_{20}F_9NO_4$ : 569,429 | | | | |
|---|---|---|---|---|
| Calculé :<br>Trouvé : | C% 52,73<br>52,8 | H% 3,54<br>3,5 | N% 2,46<br>2,5 | F% 30,03<br>30,3 |

Exemple 10 :

(1R,3S)-3-[(E)-3-(1,1-diméthyléthoxy)-2-fluoro-3-oxo-1-propényl] -2,2-diméthyl-cyclopropanecarboxylate de 1-[1-(2-propynyl)-2-trifluorométhyl-(1H)-indol-3-yl]éthyle

On opère comme à l'exemple 8 en utilisant au départ 1,06 g de 1(RS) [1-(2-propynyl) 2-trifluorométhyl (1H) indol-3-yl] éthanol et 1,033 g de l'acide approprié en maintenant le milieu réactionnel sous agitation à température ambiante pendant 22 heures. On obtient 1,41 g de produit attendu.
$[alpha]_D$ = -1,6¤ ± 1,4¤ (c = 0,6%, toluène)

| Analyse : $C_{27}H_{29}F_4NO_4$ : 507,531 | | | | |
|---|---|---|---|---|
| Calculé :<br>Trouvé : | C% 63,90<br>64,0 | H% 5,76<br>5,9 | N% 2,76<br>2,9 | F% 14,96<br>15,0 |

Préparation 4 :

Le 1(RS) [1-(2-propynyl) 2-trifluorométhyl (1H) indol-3-yl] éthanol

On refroidit à -10¤C, 3,55 g de 1-(2-propynyl) 2-trifluorométhyl 3-formyl (1H) indole dans 30 cm3 d'éther et ajoute en 20 minutes 10 cm3 d'une solution d'iodure de méthyl magnésium dans l'éther (1,6 M). On agite pendant 1 heure à -10¤/-5¤C, laisse revenir à température ambiante, agite 30 minutes et ajoute de nouveaux 2 cm3 de la solution magnésienne. On maintient sous agitation pendant 45 minutes, verse dans une solution aqueuse de chlorure d'ammonium glacée, extrait à l'éther, sèche et élimine le solvant sous pression réduite. On chromatographie le résidu sur silice (éluant : hexane-acétate d'éthyle 8-2) et obtient 3,0 g de l'alcool attendu.

Exemple 11 :

(1R,3S)-3-((E)-3-éthoxy-2-fluoro-3-oxo-1-propényl) -2,2-diméthyl-cyclopropanecarboxylate de 1-[1-(2-propynyl)-2-trifluorométhyl-(1H)-indol-3-yl]éthyle

On opère comme à l'exemple 8 en utilisant au départ 1,08 g de 1(RS) [1-(2-propynyl) 2-trifluorométhyl (1H) indol-3-yl] éthanol et de 4 cm3 de solution chlorométhylénique de l'acide approprié (M) en maintenant le milieu réactionnel sous agitation à température ambiante pendant 2 heures et demie. On obtient 1,47 g de produit attendu.
$[alpha]_D$ = +16¤ ± 1,4¤ (c = 0,7%, toluène)

| Analyse : $C_{25}H_{25}F_4NO_4$ | | | | |
|---|---|---|---|---|
| Calculé :<br>Trouvé : | C% 62,63<br>62,9 | H% 5,26<br>5,1 | N% 2,92<br>2,8 | F% 15,85<br>15,8 |

Exemple 12 :

(1R,3S)-3-((E)-3-éthoxy-2-fluoro-3-oxo-1-propényl) -2,2-diméthyl-cyclopropanecarboxylate de 1-[1-(2-propynyl)-2-trifluorométhyl-(1H)-indol-3-yl]prop-2-ynyle

On opère comme à l'exemple 8 en utilisant au départ 0,95 g d'alcool 1 (RS) [1-(2-propynyl) 2-trifluorométhyl (1H) indol-3-yl] prop-2-ynylique et 0,76 g d'acide approprié en maintenant le milieu réactionnel sous agitation à température ambiante pendant 19 heures. On obtient 1,21 g de produit attendu.
$[alpha]_D$ = -8,5¤ ± 1¤ (c = 1%, toluène)

| Analyse : $C_{26}H_{23}F_4NO_4$ : 489,472 | | | | |
|---|---|---|---|---|
| Calculé :<br>Trouvé : | C% 63,80<br>64,1 | H% 4,74<br>4,9 | N% 2,86<br>3,0 | F% 15,52<br>15,4 |

Préparation 5 :

L'alcool 1(RS) [1-(2-propynyl) 2-trifluorométhyl (1H) indol-3-yl] prop-2-ynylique a été préparé comme suit :

On refroidit à -15¤C, 4,5 g de 1-(2-propynyl) 2-trifluorométhyl 3-formyl (1H) indole dans 30 cm3 de tétrahydrofuranne et ajoute 15 minutes, 26 cm3 d'une solution de bromure d'éthynyl magnésium dans le tétrahydrofuranne (0,8 M). On agite 1 heure à -5¤C, ajoute de nouveau 5 cm3 de la solution magnésienne, laisse revenir à température ambiante, agite 30 minutes et verse dans une solution aqueuse saturée de phosphate monosodique. On extrait à l'éther isopropylique, élimine le solvant sous pression réduite, obtient 5,89 g de produit brut que l'on purifie par chromatographie sur silice (éluant : hexane-acétate d'éthyle 9-1) et obtient 1,91 g de l'alcool attendu. F = 98¤C.

Exemple 13 :

(1R,3S)-3-[(E)-3-(1,1-diméthyléthoxy)-2-fluoro-3-oxo-1-propényl] -2,2-diméthyl-cyclopropanecarboxylate de 1-[1-(2-propynyl)-2-trifluorométhyl-(1H)-indol-3-yl]prop-2-ynyle

On opère comme à l'exemple 8 en utilisant au départ 0,95 g d'alcool et 0,9 g d'acide approprié et an maintenant le milieu réactionnel sous agitation à température ambiante pendant 18 heures. On obtient 1,19 g de produit attendu.
$[alpha]_D$ = +11,5¤ ± 1¤ (c = 1%, toluène)

| Analyse : $C_{28}H_{27}F_4NO_4$ : 517,526 | | | | |
|---|---|---|---|---|
| Calculé :<br>Trouve : | C% 65,00<br>65,1 | H% 5,26<br>5,3 | N% 2,70<br>2,7 | F% 14,68<br>14,9 |

**Exemple 14 : Compositions acaricides sous forme de concentré soluble.**

On effectue un mélange homogène de :

| | |
|---|---|
| Produit de l'exemple 3 | 50 g |
| Butoxyde de pipéronyle | 100 g |
| Tween 70® | 50 g |
| Topanol A® | 5 g |
| Eau q.s.p | 1000 g |

**Exemple 15 : Compositions acaricides sous forme de concentré émulsifiable.**

On effectue un mélange homogène de produit de

| | |
|---|---|
| l'exemple 8 | 100 g |
| Atlox 4851® | 64 g |
| Atlox 4855® | 32 g |
| Xylène q.s.p | 1000 g |

**Exemple 16 : Compositions acaricides sous forme de poudre mouillable**.

On mélange de façon homogène :

| | |
|---|---|
| Produit de l'exemple 8 | 50 g |
| Butyl hydroxy toluène | 2 g |
| Glycol | 60 g |
| Polyéthoxy éther d'alcools gras | 80 g. |
| Naphtalène sulfonate de sodium | 60 g |
| Kaolin q.s.p | 1000 g. |

**Exemple 17 : Compositions insecticide fumigène**.

On mélange d'une façon homogène :

| | |
|---|---|
| Produit de l'exemple 5 | 10 g |
| Poudre de tabu | 250 g |
| Poudre de feuilles de cèdre | 390 g |
| Poudre de bois de pin | 340 g |
| Vert brillant | 5 g |
| p-nitrophénol | 5 g |

**Etude de l'activité acaricide sur Tetranychus Urticae**.

Essai adulticide.

On utilise des feuilles de haricots infestées de 50 acariens par feuilles et enduits de glu à leur périphérie.

On opère une pulvérisation de 2,5 cm3 de solution aqueuse de composé par feuille en utilisant des concentrations décroissantes.

On détermine la $CL_{50}$ des composés de l'invention.

Les résultats expérimentaux sont résumés dans le tableau suivant :

| Produit de l'exemple | $CL_{50}$ produit (mg/l) |
|---|---|
| 3 | 283 |
| 5 | 132 |
| 8 | 23 |

**Etude de l'activité insecticide sur Aphis Cracivora**.

On utilise des plants de fèves que l'on divise en deux groupes.

a) le premier groupe de plants est traité par le composé de l'invention.

On pulvérise sur chaque feuille 1 ml de solution contenant des quantités décroissantes de composé de l'invention par litre, infeste chaque feuille de vingt pucerons et entoure chaque feuille de gaze pour empêcher le départ des pucerons. On détermine la $DL_{50}$ du composé de l'invention.

b) Le deuxième groupe de plants ou groupe témoin n'est pas traité. On infeste directement chaque feuille de vingt pucerons. Les résultats expérimentaux sont résumés dans le tableau suivant :

| Produit de l'exemple | $DL_{50}$ Produit de l'invention (mg/l) |
|---|---|
| 1 | 5,7 |
| 3 | 5,7 |
| 5 | 3,4 |
| 8 | 1,5 |

## Revendications

### Revendications pour les Etats contractants suivants : BE, CH, DE, FR, GB, IT, LI, NL

**1.** Les composés de formule (I) :

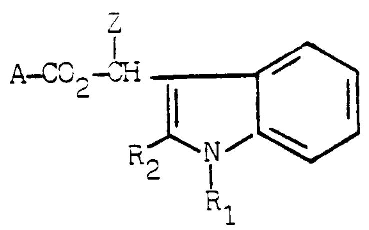

(I)

dans laquelle Z représente un atome d'hydrogène, un groupement C≡N, C≡CH, $CF_3$ ou un radical alkyle renfermant de 1 à 3 atomes de carbone, $R_1$ représente un atome d'hydrogène, un radical alkyle, linéaire, ramifié, cyclique, saturé ou insaturé renfermant jusqu'à 8 atomes de carbone, un radical phényle ou naphtyle ou un radical benzyle, phényl éthyle, phényl propyle, phényl butyle, phényl pentyle ou phényl hexyle, et $R_2$ représente un atome d'hydrogène, un atome d'halogène, un radical alkyle, linéaire, ramifié ou cyclique, saturé ou insaturé, renfermant jusqu'à 8 atomes de carbone, un radical phényle ou napthyle, un radical benzyle, phényl éthyle, phényl propyle, phényl butyle, phényl pentyle, phényl hexyle, un radical cyano, un radical $CF_3$, un radical $CO_2$alkyle renfermant jusqu'à 18 atomes de carbone, un radical $NO_2$ et A représente un radical :

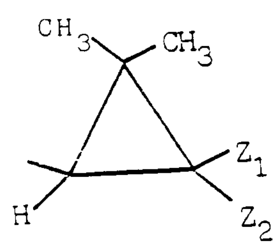

dans lequel $Z_1$ représente un atome d'hydrogène et $Z_2$ représente un radical :

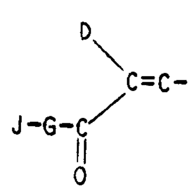

dans lequel D représente un atome d'hydrogène ou d'halogène, un radical alkoxyle renfermant de 1 à 8 atomes de carbone, G représente un atome d'oxygène ou de soufre et J représente un radical alkyle

linéaire, ramifié ou cyclique, saturé ou insaturé, renfermant de 1 à 8 atomes de carbone, éventuellement substitué par un ou plusieurs atomes d'halogène lorsque D est un atome d'hydrogène.

**2.** Les composés de formule (I), tels que définis à la revendication 1, dans lesquels $R_1$ représente un radical $-CH_2-C{\equiv}CH$.

**3.** Les composés de formule (I), tels que définis à la revendication 1, dans lesquels $R_1$ représente un radical $-CH_2-C_6H_5$.

**4.** Les composés de formule (I), tels que définis à la revendication 1, dans lesquels $R_1$ représente un atome d'hydrogène.

**5.** Les composés de formule (I), tels que définis à l'une quelconque des revendications 1 à 4 dans lesquels $R_2$ représente un radical $CF_3$.

**6.** Les composés de formule (I), tels que définis à l'une quelconque des revendications 1 à 5 dans lesquels A représente un radical :

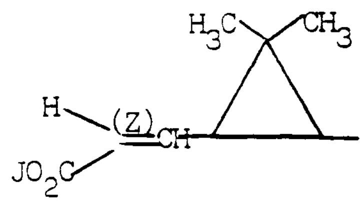

dans lequel J représente un radical alkyle renfermant de 1 à 8 atomes de carbone, linéaire, ramifié ou cyclisé et saturé éventuellement substitué par un ou plusieurs atomes d'halogène $Hal_2$, la double liaison ayant la géométrie Z.

**7.** Les composés de formule (I) tels que définis à l'une quelconque des revendications 1 à 5 dans lesquels A représente un radical :

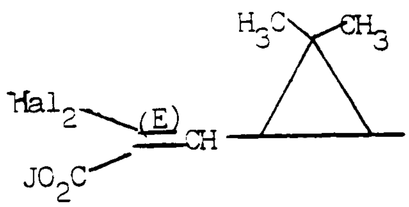

dans lequel $Hal_2$ représente un atome d'halogène et J représente un radical alkyle renfermant de 1 à 8 atomes de carbone, linéaire, ramifié ou cyclisé et saturé, la double liaison ayant la géométrie (E).

**8.** Les composés de formule (I) tels que définis à la revendication 6 ou 7 dans lesquels $Hal_2$ représente un atome de fluor.

**9.** Le produit défini à la revendication 1 dont le nom suit :
(1R,3S)-2,2-diméthyl-3-[3-oxo-3-[2,2,2-trifluoro-1-(trifluorométhyl) éthoxy]-1-propényl]-cyclopropanecarborylate de [1-(2-propynyl)-2-trifluorométhyl-(1H)-indol-3-yl] méthyle.

**10.** Procédé de préparation de composés de formule (I) tels que définis à l'une quelconque des revendications 1 à 9, caractérisé en ce que l'on soumet un alcool de formule (II) :

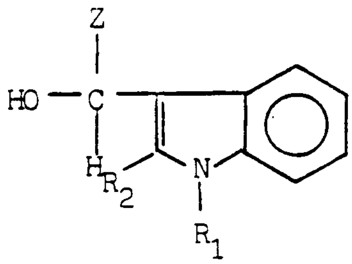

(II)

dans laquelle Z, $R_1$ et $R_2$ ont la signification indiquée précédemment à l'action d'un acide de formule (III) :

$ACO_2H$ (III)

dans laquelle A a la signification indiquée précédemment ou d'un dérivé fonctionnel de cet acide et obtient le composé de formule (I) correspondant.

**11.** Application des composés de formule (I) tels que définis à l'une quelconque des revendications 1 à 9, à la lutte contre les parasites des végétaux, les parasites des locaux et les parasites des animaux à sang chaud.

**12.** Les compositions pesticides destinées à la lutte contre les parasites des végétaux, les parasites des locaux et les parasites des animaux à sang chaud, caractérisées en ce qu'elles renferment comme principe actif au moins un des produits définis à l'une quelconque des revendications 1 à 9.

**13.** Les compositions pesticides destinées à la lutte contre les parasites des végétaux, les parasites des locaux et les parasites des animaux à sang chaud, caractérisées en ce qu'elles renferment comme principe actif le produit de la revendication 9.

**14.** Les compositions acaricides destinées à lutter contre les acariens parasites des végétaux, caractérisées en ce qu'elles renferment au moins un produit défini à l'une quelconque des revendications 1 à 9.

**15.** Les compositions insecticides renfermant comme principe actif au moins un des produits définis à l'une quelconque des revendications 1 à 9.

**16.** Les compositions nématicides renfermant comme principe actif au moins l'un des produits définis à l'une quelconque des revendications 1 à 9.

**17.** Les compositions acaricides destinées à la lutte contre les parasites des animaux à sang chaud, caractérisée en ce qu'elles renferment comme principe actif, au moins un des produits définis à l'une quelconque des revendications 1 à 9.

**18.** Associations douées d'activité insecticide, acaricide ou nématicide, caractérisées en ce qu'elles contiennent comme matière active, d'une part un au moins des composés de formule générale (I) telle que définie à la revendication 1, et d'autre part, un au moins des esters pyréthrinoïdes choisis dans le groupe constitué par les esters d'alléthrolone, d'alcool 3,4,5,6-tétrahydrophtalimido méthylique, d'alcool 5-benzyl 3-furyl benzylique des acides chrysanthémiques, par les esters d'alcool 5-benzyl 3-furyl méthylique des acides 2,2-diméthyl 3-(2-oxo 3-tétrahydrothiophénylidène méthyl) cyclopropane-1-carboxyliques, par les esters d'alcool 3-phénoxy benzylique et d'alcools alpha-cyano 3-phénoxy benzyliques des acides 2,2-diméthyl 3-(2,2-dichlorovinyl) cyclopropane-1-carboxyliques, par les esters d'alcools alpha-cyano 3-phénoxy benzyliques d'acides 2,2-diméthyl 3-(2,2-dibromovinyl) cyclopropane-1-carboxyliques, par les esters d'alcool 3-phénoxy benzylique des acides 2-parachlorophényl 2-isopropyl acétiques, par les esters d'alléthrolone, d'alcool 3,4,5,6-tétrahydrophtalimido méthylique, d'alcool 5-benzyl 3-furyl méthylique, d'alcool 3-phénoxy benzylique et d'alcools alphacyano 3-phénoxybenzyliques des acides 2,2-diméthyl 3-(1,2,2,2-tétrahaloéthyl) cyclopropane-1-carboxyliques, dans lesquels "halo" représente un atome de fluor, de chlore ou de brome, étant entendu que les copules acides et alcools des esters pyréthrinoïdes ci-dessus peuvent exister sous toutes leurs formes

stéréoisomères possibles.

**19.** Les compositions telles que définies à l'une quelconque des revendications 12 à 18, caractérisées en ce qu'elles renferment en outre un synergiste des pyréthrinoïdes.

**Revendications pour les Etats contractants suivants : ES, GR**

**1.** Procédé de préparation des composes de formule (I) :

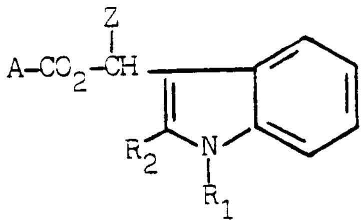

(I)

dans laquelle Z représente un atome d'hydrogène, un groupement C≡N, C≡CH, $CF_3$ ou un radical alkyle renfermant de 1 à 3 atomes de carbone, $R_1$ représente un atome d'hydrogène, un radical alkyle, linéaire, ramifié, cyclique, saturé ou insaturé renfermant jusqu'à 8 atomes de carbone, un radical phényle ou naphtyle ou un radical benzyle, phényl éthyle, phényl propyle, phényl butyle, phényl pentyle ou phényl hexyle, et $R_2$ représente un atome d'hydrogène, un atome d'halogène, un radical alkyle, linéaire, ramifié ou cyclique, saturé ou insaturé, renfermant jusqu'à 8 atomes de carbone, un radical phényle ou napthyle, un radical benzyle, phényl éthyle, phényl propyle, phényl butyle, phényl pentyle, phényl hexyle, un radical cyano, un radical $CF_3$, un radical $Co_2$alkyle renfermant jusqu'à 18 atomes de carbone, un radical $NO_2$ et A représente un radical :

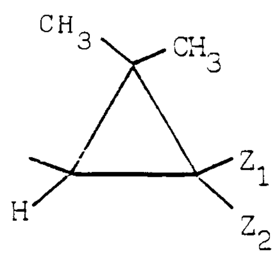

dans lequel $Z_1$ représente un atome d'hydrogène et $Z_2$ représente un radical :

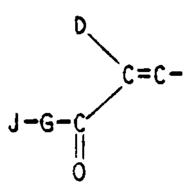

dans lequel D représente un atome d'hydrogène ou d'halogène, un radical alkoxyle renfermant de 1 à 8 atomes de carbone, G représente un atome d'oxygène ou de soufre et J représente un radical alkyle linéaire, ramifié ou cyclique, saturé ou insaturé, renfermant de 1 à 8 atomes de carbone, éventuellement substitué par un ou plusieurs atomes d'halogène lorsque D est un atome d'hydrogène, caractérisé en ce que l'on soumet un alcool de formule (II) :

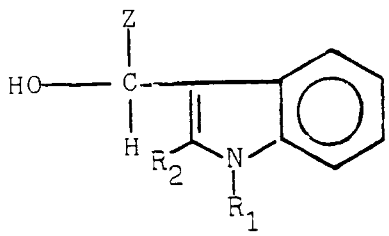

(II)

dans laquelle Z, $R_1$ et $R_2$ ont la signification indiquée précédemment à l'action d'un acide de formule (III) :

$ACO_2H$ (III)

dans laquelle A a la signification indiquée précédemment ou d'un dérivé fonctionnel de cet acide et obtient le composé de formule (I) correspondant.

**2.** Procédé selon la revendication 1, caractérisé en ce que l'on utilise au départ un alcool de formule (II) dans laquelle $R_1$ représente un radical $-CH_2-C\equiv CH$.

**3.** Procédé selon la revendication 1, caractérisé en ce que l'on utilise au départ un alcool de formule (II) dans laquelle $R_1$ représente un radical $-CH_2-C_6H_5$.

**4.** Procédé selon la revendication 1, caractérisé en ce que l'on utilise au départ un alcool de formule (II) dans laquelle $R_1$ représente un atome d'halogène.

**5.** Procédé selon l'une quelconque des revendications 1 à 4, caractérisé en ce que l'on utilise au départ un alcool de formule (II) dans laquelle $R_2$ représente un radical $CF_3$.

**6.** Procédé selon l'une quelconque des revendications 1 à 5, caractérisé en ce que l'on utilise au départ un produit de formule (III) dans laquelle A représente un radical :

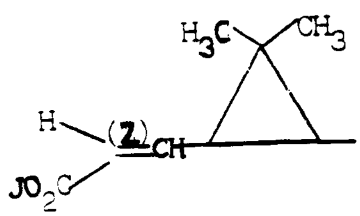

dans lequel J représente un radical alkyle renfermant de 1 à 8 atomes de carbone, linéaire, ramifié ou cyclisé, saturé éventuellement substitué par un ou plusieurs atomes d'halogène $Hal_2$, la double liaison ayant la géométrie Z.

**7.** Procédé selon l'une quelconque des revendications 1 à 5, caractérisé en ce que l'on utilise au départ un produit de formule (III) dans laquelle A représente un radical :

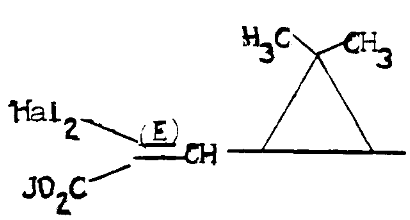

dans lequel $Hal_2$ représente un atome d'halogène et J représente un radical alkyle renfermant de 1 à 8

atomes de carbone, linéaire, ramifié ou cyclisé, saturé, la double liaison ayant la géométrie (E).

**8.** Procédé selon la revendication 6 à 7, caractérisé en ce que dans le radical A, $Hal_2$ représente un atome de fluor.

## Claims

## Claims for the following Contracting States : BE, CH, DE, FR, GB, IT, LI, NL

**1.** The compounds of formula (I):

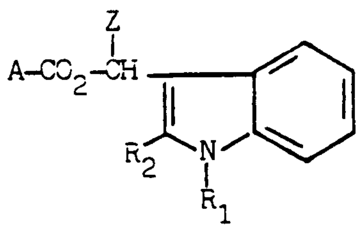

(I)

in which Z represents a hydrogen atom, a C≡N, C≡CH, $CF_3$ group or an alkyl radical containing 1 to 3 carbon atoms, $R_1$ represents a hydrogen atom, a saturated or unsaturated, linear, branched or cyclic alkyl radical containing up to 8 carbon atoms, a phenyl or naphthyl radical or a benzyl, phenyl ethyl, phenyl propyl, phenyl butyl, phenyl pentyl or phenyl hexyl radical, and $R_2$ represents a hydrogen atom, a halogen atom, a saturated or unsaturated, linear, branched or cyclic alkyl radical containing up to 8 carbon atoms, a phenyl or naphthyl radical, a benzyl, phenyl ethyl, phenyl propyl, phenyl butyl, phenyl pentyl, or phenyl hexyl radical, a cyano radical, a $CF_3$ radical, a $Co_2$alkyl radical containing up to 18 carbon atoms, an $NO_2$ radical and A represents a radical:

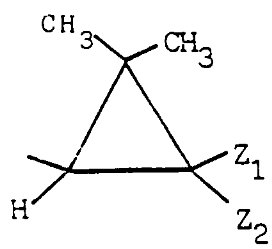

in which $Z_1$ represents a hydrogen atom and $Z_2$ represents a radical:

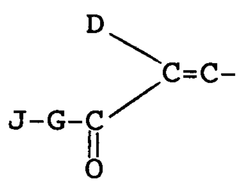

in which D represents a hydrogen or halogen atom, an alkoxyl radical containing 1 to 8 carbon atoms, G represents an oxygen or sulphur atom and J represents a saturated or unsaturated, linear, branched or cyclic alkyl radical containing 1 to 8 carbon atoms, optionally substituted by one or more halogen atoms when D is a hydrogen atom.

**2.** The compounds of formula (I), as defined in claim 1, in which $R_1$ represents a $-CH_2-C{\equiv}CH$ radical.

**3.** The compounds of formula (I), as defined in claim 1, in which $R_1$ represents a $-CH_2-C_6H_5$ radical.

**4.** The compounds of formula (I), as defined in claim 1, in which $R_1$ represents a hydrogen atom.

**5.** The compounds of formula (I), as defined in any one of claims 1 to 4 in which $R_2$ represents a $CF_3$ radical.

**6.** The compounds of formula (I), as defined in any one of claims 1 to 5 in which A represents a radical:

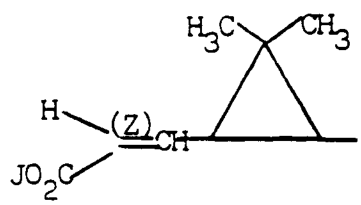

in which J represents a saturated linear, branched or cyclic alkyl radical containing 1 to 8 carbon atoms, optionally substituted by one or more halogen atoms $Hal_2$, the double bond having Z geometry.

**7.** The compounds of formula (I) as defined in any one of claims 1 to 5 in which A represents a radical:

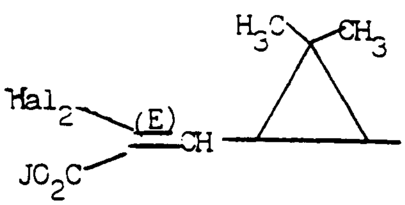

in which $Hal_2$ represents a halogen atom and J represents a saturated linear, branched or cyclic alkyl radical containing 1 to 8 carbon atoms, the double bond having (E) geometry.

**8.** The compounds of formula (I) as defined in claim 6 or 7 in which $Hal_2$ represents a fluorine atom.

**9.** The product defined in claim 1 the name of which follows:
(1R,3S) 2,2-dimethyl-3-[3-oxo-3-[2,2,2-trifluoro-1-(trifluoromethyl)-ethoxy]-1-propenyl]-cyclopropanecarboxylate of [1-(2-propynyl)-2-trifluoromethyl-(1H)-indol-3-yl]-methyl.

**10.** Preparation process for compounds of formula (I) as defined in any one of claims 1 to 9, characterized in that an alcohol of formula (II):

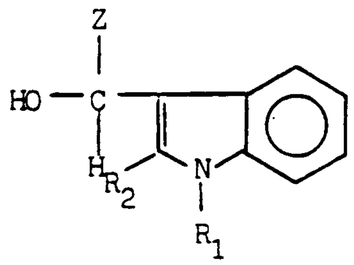

(II)

in which Z, $R_1$ and $R_2$ have meaning indicated previously, is subjected to the action of an acid of formula (III):

$ACO_2H$ (III)

in which A has the meaning indicated previously, or a functional derivative of this acid, and the corresponding compound of formula (I) is obtained.

**11.** Use of the compounds of formula (I) as defined in any one of claims 1 to 9, for combating parasites of vegetation, parasites of premises and parasites of warm-blooded animals.

**12.** The pesticide compositions intended for combating parasites of vegetation, parasites of premises and parasites of warm-blooded animals, characterized in that they contain as active ingredient at least one of the products defined in any one of claims 1 to 9.

**13.** The pesticide compositions intended for combating parasites of vegetation, parasites of premises and parasites of warm-blooded animals, characterized in that they contain as active ingredient the product of claim 9.

**14.** The Acaricide compositions intended for combating parasitic acarida of vegetation, characterized in that they contain as least one product defined in any one of claims 1 to 9.

**15.** The insecticide compositions containing as active ingredient at least one of the products defined in any one of claims 1 to 9.

**16.** The nematicide compositions containing as active ingredient at least one of the products defined in any one of claims 1 to 9.

**17.** The acaricide compositions intended for combating parasites of warm-blooded animals, characterized in that they contain as active ingredient at least one of the products defined in any one of claims 1 to 9.

**18.** Combinations endowed with insecticide, acaricide or nematicide activity, characterized in that they contain as active ingredient, on the one hand at least one of the compounds of general formula (I) as defined in claim 1, and on the other hand, at least one of the pyrethrinoid esters chosen from the group constituted by the esters of allethrone, of 3,4,5,6-tetrahydro-phthalimidomethyl alcohol, of 5-benzyl-3-furyl benzyl alcohol with chrysanthemic acids, by the esters of 5-benzyl-3-furyl methyl alcohol with 2,2-dimethyl-3-(2-oxo-3-tetrahydrothiophenylidenemethyl)-cyclopropane-1-carboxylic acids, by the esters of 3-phenoxybenzyl alcohol and of alpha-cyano-3-phenoxybenzyl alcohols with 2,2-dimethyl-3-(2,2-dichlorovinyl)-cyclopropane-1-carboxylic acids, by the esters of alphacyano-3-phenoxy-benzyl alcohols with 2,2-dimethyl-3-(2,2-dibromovinyl)-cyclopropane-1-carboxylic acids, by the esters of 3-phenoxybenzyl alcohol with 2-parachlorophenyl-2-isopropyl acetic acids, by the esters of allethrolone, of 3,4,5,6-tetrahydrophthalimidomethyl alcohol, of 5-benzyl-3-furyl methyl alcohol, of 3-phenoxybenzyl alcohol, and of alpha-cyano-3-phenoxybenzyl alcohols with 2,2-dimethyl-3-(1,2)-2,2-tetrahaloethyl)-cyclopropane-1-carboxylic acids, in which "halo" represents a fluorine,chlorine or bromine atom, it being understood that the acid and alcohol copulas of the above pyrethrinoid esters can exist in all their possible stereoisomer forms.

**19.** The compositions as defined in any one of claims 12 to 18, characterized in that they contain in addition a pyrethrinoid synergist.

**Claims for the following Contracting States : ES, GR**

**1.** Preparation process for the compounds of formula (I):

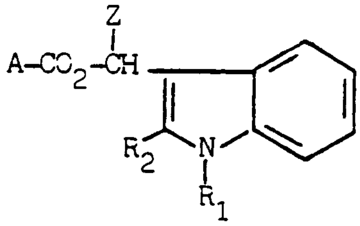

(I)

in which Z represents a hydrogen atom, a C≡N, C≡CH, $CF_3$ group or an alkyl radical containing 1 to 3 carbon atoms, $R_1$ represents a hydrogen atom, a saturated or unsaturated, linear, branched or cyclic alkyl radical containing up to 8 carbon atoms, a phenyl or naphthyl radical or a benzyl, phenyl ethyl, phenyl propyl, phenyl butyl, phenyl pentyl or phenyl hexyl radical, and $R_2$ represents a hydrogen atom, a halogen atom, a saturated or unsaturated, linear, branched or cyclic alkyl radical containing up to 8

carbon atoms, a phenyl or naphthyl radical, a benzyl, phenyl ethyl, phenyl propyl, phenyl butyl, phenyl pentyl, or phenyl hexyl radical, a cyano radical, a $CF_3$ radical, a $Co_2$alkyl radical containing up to 18 carbon atoms, an $NO_2$ radical and A represents a radical:

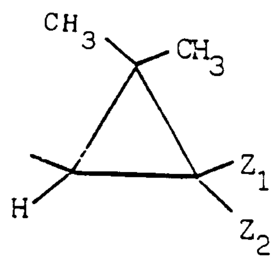

in which $Z_1$ represents a hydrogen atom and $Z_2$ represents a radical:

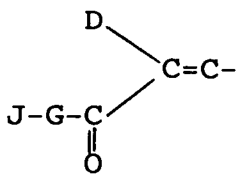

in which D represents a hydrogen or halogen atom, an alkoxyl radical containing 1 to 8 carbon atoms, G represents an oxygen or sulphur atom and J represents a saturated or unsaturated, linear, branched or cyclic alkyl radical containing 1 to 8 carbon atoms, optionally substituted by one or more halogen atoms when D is a hydrogen atom, characterized in that an alcohol of formula (II):

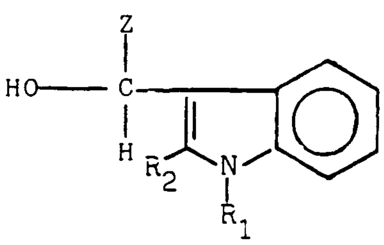

(II)

in which Z, $R_1$ and $R_2$ have the meaning indicated previously, is subjected to the action of an acid of formula (III):

$ACO_2H$ (III)

in which A has the meaning indicated previously, or a functional derivative of this acid, and the corresponding compound of formula (I) is obtained.

2. Process according to claim 1, characterized in that an alcohol of formula (II) in which $R_1$ represents a $-CH_2-C{\equiv}CH$ radical is used at the start.

3. Process according to claim 1, characterized in that an alcohol of formula (II) in which $R_1$ represents a $-CH_2-C_6H_5$ radical is used at the start.

4. Process according to claim 1, characterized in that an alcohol of formula (II) in which $R_1$ represents a halogen atom is used at the start.

5. Process according to any one of claims 1 to 4, characterized in that an alcohol of formula (II) in which $R_2$ represents a $CF_3$ radical is used at the start.

6. Process according to any one of claims 1 to 5, characterized in that a product of formula (III) is used at the start in which A represents a radical:

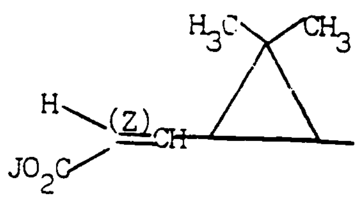

in which J represents a saturated linear, branched or cyclic alkyl radical containing 1 to 8 carbon atoms, optionally substituted by one or more halogen atoms $Hal_2$, the double bond having Z geometry.

7. Process according to any one of claims 1 to 5, characterized in that a product of formula (III) is used at the start in which A represents a radical:

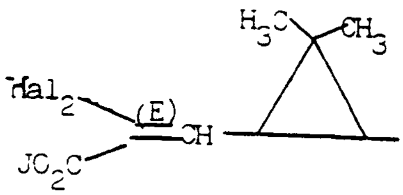

in which $Hal_2$ represents a halogen atom and J represents a saturated linear, branched or cyclic alkyl radical containing 1 to 8 carbon atoms, the double bond having E geometry.

8. Process according to claim 6 to 7, characterized in that in the radical A, $Hal_2$ represents a fluorine atom.

**Patentansprüche**
**Patentansprüche für folgende Vertragsstaaten : BE, CH, DE, FR, GB, IT, LI, NL**

1. Verbindungen der Formel (I)

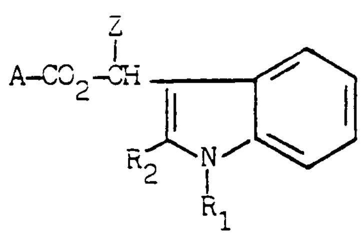

(I)

worin Z für ein Wasserstoffatom, eine Gruppe C≡N, C≡CH, $CF_3$ oder einen Alkylrest mit 1 bis 3 Kohlenstoffatomen steht, $R_1$ ein Wasserstoffatom, einen gesättigten oder ungesättigten, linearen, verzweigten oder cyclischen Alkylrest mit bis zu 8 Kohlenstoffatomen, einen Phenyl-oder Naphthylrest oder einen Benzyl-, Phenylethyl-, Phenylpropyl-, Phenylbutyl-, Phenylpentyl- oder Phenylhexylrest bedeutet und $R_2$ für ein Wasserstoffatom, ein Halogenatom, einen gesättigten oder ungesättigten, linearen, verzweigten oder cyclischen Alkylrest mit bis zu 8 Kohlenstoffatomen, einen Phenyl- oder Naphthylrest, einen Benzyl-, Phenylethyl-, Phenylpropyl-, Phenylbutyl-, Phenylpentyl-, Phenylhexylrest, eine Cyanogruppe, einen Rest $CF_3$, einen Rest $CO_2$-alkyl mit bis zu 18 Kohlenstoffatomen, eine Gruppe $NO_2$ steht und A einen Rest

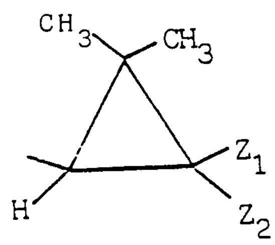

wiedergibt, worin $Z_1$ für ein Wasserstoffatom steht und $Z_2$ einen Rest

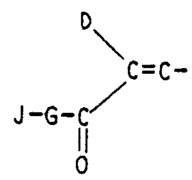

bedeutet, worin D ein Wasserstoff- oder Halogenatom, einen Alkoxyrest mit 1 bis 8 Kohlenstoffatomen wiedergibt, G für ein Sauerstoff- oder Schwefelatom steht und J einen gesättigten oder ungesättigten, linearen, verzweigten oder cyclischen Alkylrest mit 1 bis 8 Kohlenstoffatomen bedeutet, welcher gegebenenfalls durch ein oder mehrere Halogenatome substituiert ist, wenn D ein Wasserstoffatom bedeutet.

2. Verbindungen der Formel (I) gemäß Anspruch 1, worin $R_1$ für einen Rest $-CH_2-C{\equiv}CH$ steht.

3. Verbindungen der Formel (I) gemäß Anspruch 1, worin $R_1$ für einen Rest $-CH_2-C_6H_5$ steht.

4. Verbindungen der Formel (I) gemäß Anspruch 1, worin $R_1$ für ein Wasserstoffatom steht.

5. Verbindungen der Formel (I) gemäß einem der Ansprüche 1 bis 4, worin $R_2$ für einen Rest $CF_3$ steht.

6. Verbindungen der Formel (I) gemäß einem der Ansprüche 1 bis 5, worin A für einen Rest

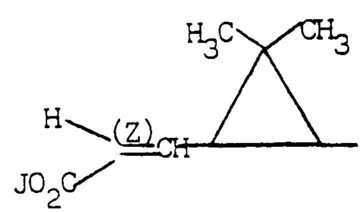

steht, worin J einen gesättigten, linearen, verzweigten oder cyclischen Alkylrest mit 1 bis 8 Kohlenstoffatomen bedeutet, der gegebenenfalls durch ein oder mehrere Halogenatome $Hal_2$ substituiert ist, wobei die Doppelbindung die Z-Geometrie aufweist.

7. Verbindungen der Formel (I) gemäß einem der Ansprüche 1 bis 5, worin A für einen Rest

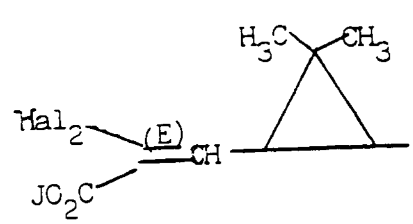

steht, worin $Hal_2$ ein Halogenatom bedeutet und J einen linearen, verzweigten oder cyclischen und gesättigten Alkylrest mit 1 bis 8 Kohlenstoffatomen bedeutet, wobei die Doppelbindung die (E)-Geometrie besitzt.

8. Verbindungen der Formel (I) gemäß Anspruch 6 oder 7, worin $Hal_2$ für ein Fluoratom steht.

9. Produkt, wie in Anspruch 1 definiert, mit der folgenden Bezeichnung:
[1-(2-Propinyl)-2-trifluormethyl-(1H)-indol-3-yl]-methyl-(1R,3S)-2,2-dimethyl-3-[3-oxo-3-[2,2,2-trifluor-1-(trifluormethyl)-ethoxy]-1-propenyl]-cyclopropancarboxylat.

10. Verfahren zur Herstellung der Verbindungen der Formel (I) gemäß einem der Ansprüche 1 bis 9, dadurch gekennzeichnet, daß man einen Alkohol der Formel (II)

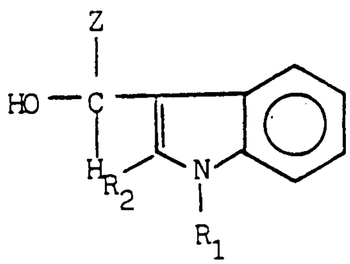

(II)

worin Z, $R_1$ und $R_2$ die vorstehend angegebene Bedeutung besitzen, der Einwirkung einer Säure der Formel (III)

$ACO_2H$ (III)

worin A die vorstehend angegebene Bedeutung besitzt, oder eines funktionellen Derivats dieser Säure unterzieht und die entsprechende Verbindung der Formel (I) gewinnt.

11. Verwendung der Verbindungen der Formel (I) gemäß einem der Ansprüche 1 bis 9 zur Bekämpfung von Parasiten der Pflanzen, Parasiten von Räumlichkeiten und Parasiten warmblütiger Tiere.

12. Pestizide Zusammensetzungen für die Bekämpfung von Parasiten der Pflanzen, Parasiten von Räumlichkeiten und Parasiten warmblütiger Tiere, dadurch gekennzeichnet, daß sie als Wirkstoff zumindest eines der Produkte gemäß einem der Ansprüche 1 bis 9 enthalten.

13. Pestizide Zusammensetzungen für die Bekämpfung von Parasiten der Pflanzen, Parasiten von Räumlichkeiten und Parasiten warmblütiger Tiere, dadurch gekennzeichnet, daß sie als Wirkstoff das Produkt gemäß Anspruch 9 enthalten.

14. Akarizide Zusammensetzungen für die Bekämpfung von parasitären Milben der Pflanzen, dadurch gekennzeichnet, daß sie zumindest ein Produkt gemäß einem der Ansprüche 1 bis 9 enthalten.

15. Insektizide Zusammensetzungen, enthaltend als Wirkstoff zumindest eines der Produkte gemäß einem der Ansprüche 1 bis 9.

16. Nematizide Zusammensetzungen, enthaltend als Wirkstoff zumindest eines der Produkte gemäß einem der Ansprüche 1 bis 9.

17. Akarizide Zusammensetzungen für die Bekämpfung von Parasiten warmblütiger Tiere, dadurch gekennzeichnet, daß sie als Wirkstoff zumindest eines der Produkte gemäß einem der Ansprüche 1 bis 9 enthalten.

18. Assoziationen mit insektizider, akarizider oder nematizider Aktivität, dadurch gekennzeichnet, daß sie als Wirkstoff einesteils zumindest eine der Verbindungen der allgemeinen Formel (I), wie in Anspruch 1 definiert, und anderenteils zumindest einen der Pyrethrinoidester, ausgewählt unter den Estern des

Allethrolons, des 3,4,5,6-Tetrahydrophthalimido-methylalkohols, des 5-Benzyl-3-furylbenzylalkohols der Chrysanthemumsäuren, unter den Estern des 5-Benzyl-3-furylmethylalkohols der 2,2-Dimethyl-3-(2-oxo-3-tetrahydrothiophenylidenmethyl)-cyclopropan-1-carbonsäuren, unter den Estern des 3-Phenoxybenzylalkohols und der α-Cyano-3-phenoxybenzylalkohole der 2,2-Dimethyl-3-(2,2-dichlorvinyl)-cyclopropan-1-carbonsäuren, unter den Estern der α-Cyano-3-phenoxybenzylalkohole der 2,2-Dimethyl-3-(2,2-dibromvinyl)-cyclopropan-1-carbonsäuren, unter den Estern des 3-Phenoxybenzylalkohols der 2-p-Chlorphenyl-2-isopropylessigsäuren, unter den Estern des Allethrolons, des 3,4,5,6-Tetrahydrophthalimido-methylalkohols, des 5-Benzyl-3-furylmethylalkohols, des 3-Phenoxybenzylalkohols und der α-Cyano-3-phenoxybenzylalkohole der 2,2-Dimethyl-3-(1,2,2,2-tetrahaloethyl)-cyclopropan-1-carbonsäuren, enthalten, worin "halo" ein Fluor-, Chlor- oder Bromatom bedeutet und wobei es sich versteht, daß die Säure- und Alkoholverknüpfungen der vorstehenden Pyrethrinoidester in sämtlichen ihrer möglichen stereoisomeren Formen vorliegen können.

**19.** Zusammensetzungen gemäß einem der Ansprüche 12 bis 18, dadurch gekennzeichnet, daß sie außerdem einen Synergisten der Pyrethrinoide enthalten.

**Patentansprüche für folgende Vertragsstaaten : ES, GR**

**1.** Verfahren zur Herstellung der Verbindungen der Formel (I)

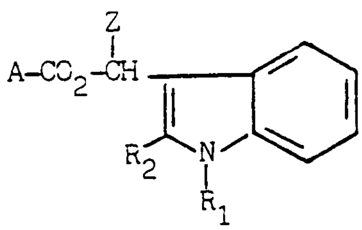

(I)

worin Z für ein Wasserstoffatom, eine Gruppe C≡N, C≡CH, $CF_3$ oder einen Alkylrest mit 1 bis 3 Kohlenstoffatomen steht, $R_1$ ein Wasserstoffatom, einen gesättigten oder ungesättigten, linearen, verzweigten oder cyclischen Alkylrest mit bis zu 8 Kohlenstoffatomen, einen Phenyl-oder Naphthylrest oder einen Benzyl-, Phenylethyl-, Phenylpropyl-, Phenylbutyl-, Phenylpentyl- oder Phenylhexylrest bedeutet und $R_2$ für ein Wasserstoffatom, ein Halogenatom, einen gesättigten oder ungesättigten, linearen, verzweigten oder cyclischen Alkylrest mit bis zu 8 Kohlenstoffatomen, einen Phenyl- oder Naphthylrest, einen Benzyl-, Phenylethyl-, Phenylpropyl-, Phenylbutyl-, Phenylpentyl-, Phenylhexylrest, eine Cyanogruppe, einen Rest $CF_3$, einen Rest $CO_2$-alkyl mit bis zu 18 Kohlenstoffatomen, eine Gruppe $NO_2$ steht und A einen Rest

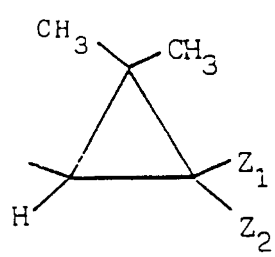

wiedergibt, worin $Z_1$ für ein Wasserstoffatom steht und $Z_2$ einen Rest

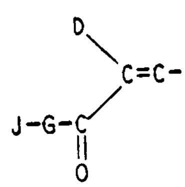

bedeutet, worin D ein Wasserstoff- oder Halogenatom, einen Alkoxyrest mit 1 bis 8 Kohlenstoffatomen wiedergibt, G für ein Sauerstoff- oder Schwefelatom steht und J einen gesättigten oder ungesättigten, linearen, verzweigten oder cyclischen Alkylrest mit 1 bis 8 Kohlenstoffatomen bedeutet, welcher gegebenenfalls durch ein oder mehrere Halogenatome substituiert ist, wenn D ein Wasserstoffatom bedeutet, dadurch gekennzeichnet, daß man einen Alkohol der Formel (II)

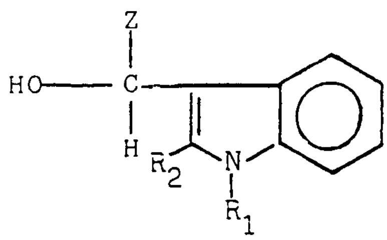

(II)

worin Z, $R_1$ und $R_2$ die vorstehend angegebene Bedeutung besitzen, der Einwirkung einer Säure der Formel (III)

$ACO_2H$ (III)

worin A die vorstehend angegebene Bedeutung besitzt, oder eines funktionellen Derivats dieser Säure unterzieht und die entsprechende Verbindung der Formel (I) gewinnt.

**2.** Verfahren gemäß Anspruch 1, dadurch gekennzeichnet, daß man von einem Alkohol der Formel (II) ausgeht, worin $R_1$ für einen Rest $-CH_2-C{\equiv}CH$ steht.

**3.** Verfahren gemäß Anspruch 1, dadurch gekennzeichnet, daß man von einem Alkohol der Formel (II) ausgeht, worin $R_1$ für einen Rest $-CH_2-C_6H_5$ steht.

**4.** Verfahren gemäß Anspruch 1, dadurch gekennzeichnet, daß man von einem Alkohol der Formel (II) ausgeht, worin $R_1$ für ein Halogenatom steht.

**5.** Verfahren gemäß einem der Ansprüche 1 bis 4, dadurch gekennzeichnet, daß man von einem Alkohol der Formel (II) ausgeht, worin $R_2$ für einen Rest $CF_3$ steht.

**6.** Verfahren gemäß einem der Ansprüche 1 bis 5, dadurch gekennzeichnet, daß man von einem Produkt der Formel (III) ausgeht, worin A einen Rest

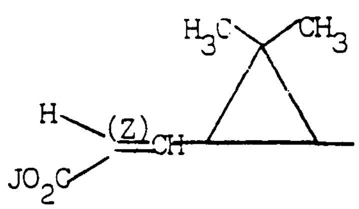

bedeutet, worin J einen gesättigten, linearen, verzweigten oder cyclischen Alkylrest mit 1 bis 8 Kohlenstoffatomen wiedergibt, der gegebenenfalls durch ein oder mehrere Halogenatome $Hal_2$ substituiert ist, wobei die Doppelbindung die Z-Geometrie aufweist.

**7.** Verfahren gemäß einem der Ansprüche 1 bis 5, dadurch gekennzeichnet, daß man von einem Produkt der Formel (III) ausgeht, worin A einen Rest

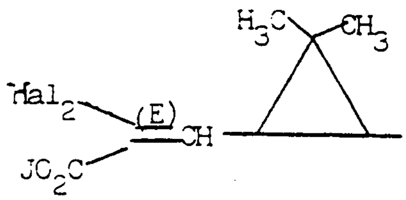

wiedergibt, worin $Hal_2$ für ein Halogenatom steht und J einen gesättigten, linearen, verzweigten oder cyclischen Alkylrest mit 1 bis 8 Kohlenstoffatomen bedeutet, wobei die Doppelbindung die (E)-Geometrie besitzt.

**8.** Verfahren gemäß Anspruch 6 bis 7, dadurch gekennzeichnet, daß in dem Rest A $Hal_2$ für ein Fluoratom steht.